# EUROPEAN PATENT SPECIFICATION

(11) **EP 2 071 321 B1**
(45) Date of publication and mention of the grant of the patent: **15.06.2011**
(21) Application number: 08021622.9
(22) Date of filing: 12.12.2008
(51) Int. Cl.: G01N 21/55, G01N 33/543

(54) **Surface plasmon resonance biosensor**
Oberflächenplasmonresonanz-Biosensor
Biocapteur à résonance à plasmons de surface

(30) Priority: 13.12.2007 JP 2007322636
(43) Date of publication of application: 17.06.2009
(73) Proprietor: FUJIFILM Corporation, Tokyo 106-0031 (JP)
(72) Inventor: Yamashita, Seiji, Ashigarakami-gun, Kanagawa (JP)
(74) Representative: HOFFMANN EITLE

(56) References cited:
- EP-A- 0 286 195
- US-A- 5 858 799
- US-A1- 2005 249 451
- WU S Y ET AL: "Sensitivity improvement of the surface plasmon resonance optical sensor by using a gold-silver transducing layer" ELECTRON DEVICES MEETING, 2002. PROCEEDINGS. 2002 IEEE HONG KONG 22 JUNE 2002, PISCATAWAY, NJ, USA,IEEE, 1 January 2002 (2002-01-01), pages 63-68, XP010600496 ISBN: 978-0-7803-7429-4
- ANUJ K SHARMA ET AL: "Fibre-optic sensor based on surface plasmon resonance with Ag-Au alloy nanoparticle films" NANOTECHNOLOGY, IOP, BRISTOL, GB, vol. 17, no. 1, 14 January 2006 (2006-01-14), pages 124-131, XP020104169 ISSN: 0957-4484
- X-C YUAN ET AL: "Sensitivity-stability-optimized surface plasmon resonance sensing with double metal layers" JOURNAL OF OPTICS. A, PURE AND APPLIED OPTICS, INSTITUTE OF PHYSICS PUBLISHING, BRISTOL, GB, vol. 8, no. 11, 1 November 2006 (2006-11-01), pages 959-963, XP020108341 ISSN: 1464-4258
- BENJAMIN BLONDER MENTORS ET AL: "Sensing Applications of Surface Plasmon Resonances" INTERNET CITATION, [Online] 19 August 2005 (2005-08-19), pages 1-11, XP007907955 Retrieved from the Internet: URL:http://eduprograms.seas.harvard.edu/re u05_papers/Blonder_Benjamin.pdf> [retrieved on 2009-03-26]
- BRUZZONE ET AL: "Comparative study of near and far field e.m. scattering response by Au/Ag alloy and core-shell nanoparticles" MATERIALS SCIENCE AND ENGINEERING C, ELSEVIER SCIENCE S.A, CH, vol. 27, no. 5-8, 8 August 2007 (2007-08-08), pages 1015-1021, XP022190432 ISSN: 0928-4931
- ROY R K ET AL: "Effect of interfacial alloying on the surface plasmon resonance of nanocrystalline Au-Ag multilayer thin films" THE EUROPEAN PHYSICAL JOURNAL B ; CONDENSED MATTER PHYSICS, EDP SCIENCES, LE, vol. 33, no. 1, 1 May 2003 (2003-05-01), pages 109-114, XP007907962 ISSN: 1434-6036
- XUAN WANG ET AL: "Electronic Dephasing in Bimetallic Gold-Silver Nanoparticles Examined by Single Particle Spectroscopy" JOURNAL OF PHYSICAL CHEMISTRY. B (ONLINE), AMERICAN CHEMICAL SOCIETY, COLUMBUS, OH, US, vol. 109, 1 January 2005 (2005-01-01), pages 20324-20330, XP007907961 ISSN: 1520-5207 [retrieved on 2005-11-10]
- LINK S ET AL: "Alloy formation of gold-silver nanoparticles and the dependence of the plasmon absorption on their composition" JOURNAL OF PHYSICAL CHEMISTRY. B, MATERIALS, SURFACES, INTERFACES AND BIOPHYSICAL, WASHINGTON, DC, US, vol. 103, no. 18, 16 April 1999 (1999-04-16), pages 3529-3533, XP002363334 ISSN: 1089-5647

## Description

### BACKGROUND OF THE INVENTION

### Field of the Invention

The present invention relates to a biosensor.

### Description of the Related Art

Recently, a large number of measurements using intermolecular interactions such as immune responses are being carried out in clinical tests, etc. Among them, several techniques are preferably used that are capable of detecting the change in the binding amount of a test substance with high sensitivity. Examples of such technique may include a surface plasmon resonance (SPR) measurement technique, a quartz crystal microbalance (QCM) measurement technique, and a measurement technique o using functional surfaces ranging from gold colloid particles to ultra-fine particles.
The SPR measurement technique utilizes the phenomenon that, in the light entering a metal film of a chip under total reflection conditions, near-field light leaking in the metal film and the outside thereof is absorbed by a surface plasmon generated on the metal film and thus an absorption peak assignable to it appears. When a biomolecule binds to an organic functional film being in contact on the metal film, the dielectric constant changes in the surrounding field. As a result, the wave number vector of the light that can excite the surface plasmon alters, which results in a shift in the reflection angle of an absorption peak appearing in the reflected light or a change in the reflected light amount at a definite reflection angle. The SPR measurement technique is a method in which such a shift or change as described above is measured and determined to thereby detect the absorption and desorption occurring in the vicinity of the surface. It is known as a method of highly sensitively detecting a specific interaction (binding) between a physiologically active substance immobilized on a gold film and another test substance.

A commonly used measurement chip comprises a transparent substrate (e.g., glass), an evaporated metal film, and a thin film having thereon a functional group capable of immobilizing a physiologically active substance. The measurement chip immobilizes the physiologically active substance on the metal surface via the functional group. A specific binding reaction between the physiological active substance and a test substance is measured, so as to analyze an interaction between biomolecules.
Examples of such a measurement chip include those having a film of a free-electron metal selected from the group consisting of copper, silver, aluminum and gold with a hydrogel such as carboxymethyl-denatured dextran and the like (see, for example, Japanese Patent No:2815120).
As described above, such a measurement chip allows the three-dimensional immobilization of a physiologically active substance having amino group thereon, which makes it highly useful as detection surface of a biosensor.

Since the SPR method depends on the principle of resonance absorption of light by surface plasmon as discussed above, a metal film having a high electrical conductivity, i.e., having a high free electron concentration is preferably used therein. Among the near-filed light leaking in the metal film and the outside thereof, the light component from the light entering at a specific angle transmitted on the metal film surface under the boundary conditions appears as a larger and sharper resonance absorption line in the reflected light due to the absorption by the surface plasmon at the higher electrical conductivity of the film. As a result, even a minor angle change can cause a large change in light amount and thus elevate the detection sensitivity. As an indication of the sharpness, the half-width of absorption line is usable.

Gold, silver, copper, platinum, aluminum, and the like have been known as metals having a high electrical conductivity. Among them, silver has been known as the metal having the highest electrical conductivity.
However, silver is active to atmospheric oxygen and moisture. Because of these properties, it is easily oxidized and cannot maintain a high stability. Therefore, it has been considered that silver is merely usable in the laboratory but unsuitable for the application to industrial purposes such as drug discovery and screening. Thus, it has been a common practice to use films made of gold that is relatively stable against oxidation though being inferior in sensitivity to silver because of the lower electrical conductivity.
Wu and Lo: "Sensitivity improvement of the surface plasmon resonance optical sensor by using a gold-silver transducing layer", Procs. Electron Devices Meeting 2002, pp. 63-68, investigates composite silver/gold films for the transducing layer of surface plasmon resonance optical sensors.
US-2005/249451-A1 discloses plasmon metal waveguides consisting of silver, platinum, gold or alloys thereof.
In document Sharma and Gupta: "Fibre-optic sensor based on surface plasmon resonance with Ag-Au alloy nanoparticle films", Nanotechnology, vol. 17, no. 1, pp. 124-131, the design of a fibre optic surface plasmon resonance sensor based on a silver-gold alloy nanoparticle film is studied.

### SUMMARY OF THE INVENTION

The present invention has been made in view of the above circumstances and provides a biosensor.

A first aspect of the invention provides A biosensor for detecting the interaction of a physiologically active substance on a substrate based on surface plasmon resonance, wherein the substrate has a metal film comprising: approximately 90% by mol or more and less than approximately 99.95% by mol of a first metal element which is Ag; and approximately 0.05% by mol or more and less than approximately 10% by mol of a second metal element, which is at least one of Au, Pt, Cu, Bi, Nd, Ti or Sb.
A second aspect of the invention provides a biosensor according to the first aspect, comprising: a detection unit comprising the metal film and the physiologically active substance which is provided on the metal film surface as a sensing substance; a supply unit for supplying a candidate substance to the detection unit; and a screening evaluation unit that evaluates whether the candidate substance is a target substrate to be screened or not based on whether or not the sensing substance and the candidate substance bind together.

### BRIEF DESCRIPTION OF THE DRAWINGS

Fig. 1 is an exploded perspective view schematically showing the constitution of a measurement chip according to an embodiment of the present invention.
Fig. 2 is an exploded perspective view schematically showing the constitution of a biosensor according to an embodiment of the invention.

### DETAILED DESCRIPTION OF THE INVENTION

The biosensor according to the present invention is a biosensor for detecting the interaction of substances on a substrate based on surface plasmon resonance, the substrate having a metal film including approximately 90% by mol or more and less than approximately 99.95% by mol of Ag as a first metal element, and approximately 0.05% by mol or more and less than approximately 10% by mol of at lease one of Bi, Nd or Sb as a second metal element.
Upon achieving the invention, it was discovered that a biosensor using a metal film comprising Ag that has a high electrical conductivity as the first metal element, together with a trace amount of at least one of Bi, Nd or Sb as the second metal element, can detect interaction between substances with high sensitivity, has improved stability and can be repeatedly used compared with a biosensor that uses Ag alone.
According to the invention, it is possible to provide a biosensor which can detect the interaction between substances with high sensitivity and can be stably and repeatedly used.

Additionally, in the invention, the term "step" indicates not only an independent step but may also indicate a step which cannot be discriminated clearly from other steps, as long as the intended effects of the step may be obtained.
Further, any notation for expressing numerical ranges in the invention indicates a range defined by the minimum and maximum values and includes the minimum and maximum values.
The term "biologically active substance" as used in the invention indicates a substance which relates to a living organism and indicates any substance that may exhibit physiological functions in vivo.
The invention will be described below.

The biosensor of the present invention has as broad a meaning as possible, and the term "biosensor" is used herein to mean a sensor, which converts an interaction between biomolecules into a signal such as an electric signal, so as to measure or detect a target substance. A conventional biosensor is comprised of a receptor site for recognizing a chemical substance as a detection target and a transducer site for converting a physical change or chemical change generated at the site into an electric signal. In a living body, there exist substances having an affinity with each other, such as enzyme/substrate, enzyme/coenzyme, antigen/antibody, and hormone/receptor. A biosensor operates on the principle that a substance having an affinity with another substance, as described above, is immobilized on a substrate to be used as a molecule-recognizing substance, so that the corresponding substance can be selectively measured.

In the biosensor according to the invention, the metal film, which is provided on the substrate, comprises approximately 90% by mol or more and less than approximately 99.95% by mol of Ag as a first metal element, and approximately 0.05% by mol or more and less than approximately 10% by mol of at least one of Bi, Nd or Sb as a second metal element.

Ag, as the first metal element, is the main component of the metal film and can contribute mainly to an improvement in sensitivity. The second metal element, which is one of Bi, Nd or Sb, is a trace metal component contained in the metal film of the invention. When combined with the first metal element, the second metal element contributes to the stabilization and maintenance of the effect due to Ag. Provided the content of the second metal element falls within the range as defined above, degradation of the first metal element caused by oxidation and the like can be effectively prevented without lowering sensitivity.
Considering sensitivity as a biosensor, it is preferable that the metal film comprises approximately 95% by mol or more and less than approximately 99.9% by mol of the first metal element and approximately 0.1% by mol or more and less than approximately 5.0% by mol of the second metal element.

Further considering sensitivity as a biosensor, it is preferable that the second metal element comprises a combination of at least one selected from the first group consisting of Au, Cu, Pt and Ti with at least one selected from the second group consisting of Bi, Nd and Sb. Au, Cu, Pt and Ti are each highly conductive metallic materials exhibiting high electrical conductivity and excellent corrosion resistance. If Au, Pt, Ti or a combination of two or more of the same are used, the oxygen-resisting properties of the metal film can be further improved.

When a metal element of a second group including Bi, Nd and Sb is employed alone, the content thereof is preferably from approximately 0.1% by mol to approximately 2% by mol. When a combination of a metal element of the first group with a metal element of the second group is employed, it is preferable that the total combined amount thereof is adjusted to be within the preferable ranges of the second metal element as described above.

With regard to the second group, when mixed with Ag, Bi, Nd or Sb frequently localizes around the surface and, as a result, lowers surface energy and effectively prevents Ag from oxidation. From the second group, Nd or Bi are particularly preferred. '

Between the metal film and the substrate described hereinafter, an intermediate layer made of, for example, chromium may be provided by taking the adhesiveness to the substrate into consideration.

The thickness of the metal film is not limited, it is preferably approximately 0.1 nm or more and approximately 500 nm or less, more preferably approximately 1 nm or more and approximately 200 nm or less. When the film thickness is approximately 500 nm or less, the surface plasmon phenomenon of a medium can be sufficiently detected. In the case of forming a protective layer comprising the second metal element, the thickness of the layer comprising the first metal element alone may be appropriately adjusted so that the total metal film thickness falls within the range as defined above. In the case of forming an intermediate layer comprising chromium or the like, the thickness of the intermediate layer is preferably approximately 0.1 nm or more and approximately 10 nm or less.

The biosensor according to the invention may have at least one of a metal oxide protective layer and a metal nitride protective layer on the metal film as described above. Specific examples of the metal to be used in the metal oxide layer or the metal nitride layer include Al, Si, Ge, In, Sn, Nb, Fe, Zn, Ga and so on. Among all, Si is particularly preferred. Although the thickness of the metal oxide layer or the metal nitride layer is not limited, it is preferably approximately 1 nm or more and approximately 50 nm or less, more preferably approximately 1 nm or more and approximately 20 nm or less.

Formation of a metal film may be carried out by common methods, and examples of such a method may include sputtering method, evaporation method, ion plating method, electroplating method, and nonelectrolytic plating method, for example, the sputtering method of metal under oxygen atmosphere may be preferably used.

A metal film is preferably placed on a substrate. The description "placed on a substrate" is used herein to mean a case where a metal film is placed on a substrate such that it directly comes into contact with the substrate, as well as a case where a metal film is placed via another layer without directly coming into contact with the substrate.
When a substrate used in the present invention is used for a surface plasmon resonance biosensor, examples of such a substrate may include, generally, optical glasses such as BK7, and synthetic resins. More specifically, materials transparent to laser beams, such as polymethyl methacrylate, polyethylene terephthalate, polycarbonate or a cycloolefin polymer, may be used. Supposing a system for screening a test compound, the base unit should act as an optical unit such as a prism and has a low cost. Thus, a plastic substrate is preferred. It is still preferred that the substrate is made of a material which shows no anisotropy for polarized light and has an excellent workability. More specifically, it is preferably used a cycloolefin polymer or the like.

The biosensor according to the invention has a layer for immobilizing a physiologically active substance on the metal film. It is preferable that the layer for immobilizing a physiologically active substance comprises at least one layer selected from among a self-assembled monolayer and a hydrophilic polymer layer. It is more preferable that the layer comprises both of these layers. Examples of the self-assembled monolayer may include an organic alkanethiol layer having at least one of an amino group and a hydroxyl group. As the hydrophilic polymer layer, a hydrophilic polymer having an activated carboxyl group is preferable from the viewpoint of the ability to immobilize the physiologically active substance.

In the invention, the metal film may be coated with the organic alkanethiol layer having at least one of an amino group and a hydroxyl group and then the organic alkanethiol layer may be reacted with the hydrophilic polymer having an activated carboxyl group.
Thus, a hydrogel as a polymer layer capable of immobilizing the physiologically active substance may be conveniently prepared.

In the invention, as the method of coating the metal film with the organic alkanethiol layer having at least one of an amino group and a hydroxyl group, a method known in the art may be employed, in view of convenience of the procedures, a coating method using self-assembled monolayers (SAMs) is preferred.
A method of coating metal films with self-assembled monolayers (SAMs) has been intensively studied by Professor Whitesides at Harvard University, and the details thereof are described in Chemical Review, 105, 1103-1169 (2005), for example.
When silver is used as metal, an alkanethiol derivative represented by formula (1) (wherein n represents an integer from 3 to 20, and X represents a functional group) is used as an organic layer-forming compound, so that a monomolecular film having orientation is formed in a self-assemble manner, based on an Ag-S bond and the van der Waals force between alkyl chains. A self-assembled monolayer can be formed by an extremely convenient procedure of immersing a metal substrate in a solution of an alkanethiol derivative. By forming a self-assembled monolayer using a compound of the formula 1 wherein X is NH₂ or OH, it is possible to coat a metal surface with an organic alkanethiol layer having an amino group and/or a hydroxyl group.

HS(CH₂)ₙX (1)

Alkanethiol having at least one of an amino group and a hydroxyl group can also form a self-assembled monolayer by itself. Otherwise, such alkanethiol can be mixed with another alkanethiol, so as to form a self-assembled monolayer. When such an alkanethiol is used as a surface of the immobilization substrate for a biosensor, a compound capable of suppressing non-specific adsorption of a physiologically active substance is preferably used as another alkanethiol. Such a self-assembled monolayer capable of suppressing non-specific adsorption of a physiologically active substance has been studied in detail by the aforementioned Professor Whitesides et al. Professor Whitesides et al. have reported that a self-assembled monolayer formed from alkanethiol having a hydrophilic group is effective for suppression of non-specific adsorption (Langrnuir, 17, 2841-2850, 5605-5620, 6336-6343 (2001)). As alkanethiol forming a mixed monomolecular film together with alkanethiol having at least one of an amino group and a hydroxyl group, a compound described in the aforementioned article may preferably used.

In the case where an alkanethiol having at least one of an amino group and a hydroxyl group is mixed with an alkanethiol having a hydrophilic group in the invention, the alkanethiol having at least one of an amino group and a hydroxyl group is mixed at an any ratio. From the viewpoint of reducing the ability to inhibit nonspecific adsorption, the mixing ratio of the alkanethiol having at least one of an amino group and a hydroxyl group with the alkanethiol having a hydrophilic group preferably ranges from 1/1 to 1/1,000,000, more preferably from 1 to 1 to 1/1,000 and still more preferably from 1 to 1/10. From the viewpoint of a reduction in steric hindrance occurring during a reaction with a polymer containing an active esterified carboxyl group, the molecular length of alkanethiol having an amino group is preferably longer than that of alkanethiol having a hydrophilic group.

As alkanethiol described above, a compound synthesized based on the summary of Professor Grzybowski at Northwestern University (Curr. Org. Chem., 8, 1763-1797 (2004)) and the cited documents thereof may be used, or a commercially available compound may also be used. Such compounds are available from Dojindo Laboratories, Aldrich, SensoPath Technologies, Frontier Scientific Inc., etc.

Examples of hydrophilic polymers which can be used in the invention may include, gelatin, agarose, chitosan, dextran, carrageenan, alginic acid, starch, cellulose or the derivatives thereof such as carboxymethyl derivative, water-swellable organic polymers such as polyvinyl alcohol, polyacrylic acid, polyacrylamide, polyethylene glycol and the derivatives thereof.

Further examples of the hydrophilic polymer usable in the invention include a synthetic polymer having a carboxyl group and a polysaccharide having a carboxyl group. Examples of the synthetic polymer having a carboxyl group include polyacrylic acid, polymethacrylic acid and copolymers thereof, a methacrylic acid copolymer, an acrylic acid copolymer, an itaconic acid copolymer, a crotonic acid copolymer, a maleic acid copolymer, a partially esterified maleic acid copolymer and a hydroxyl group-containing polymer having an acid anhydride added thereto which are disclosed, for example, in JP-B No. 54-34327, page 3, right column, 11th line from the bottom to page 4, left column, line 7; JP-B No. 58-12577, page 3, left column, line 1 to page 4, left column, line 1; JP-B No. 54-25957, page 2, right column, 2nd line from the bottom to page 4, left column, 8th line from the bottom; JP-A No. 59-53836, page 3, upper right column, line 2 to page 6, lower left column, line 9; and JP-A No. 59-71048, page 3, lower left column, line 1 to page 5, upper left column, line 3. Such a carboxyl group-containing polysaccharide may be any one selected from an extract from natural plants, a product obtained by fermentation by microorganisms, a synthetic product obtained by enzymes, and a chemically synthetic product. Specific examples of such a carboxyl group-containing polysaccharide may include hyaluronic acid, chondroitin sulfuric acid, heparin, dermatan sulfate, carboxymethyl cellulose, carboxyethyl cellulose, cellouronic acid, carboxymethyl chitin, carboxymethyl dextran, and carboxymethyl starch, and the like. As a carboxyl group-containing polysaccharide, a commercially available product can be used. Specific examples of such a carboxyl group-containing polysaccharide may include CMD, CMD-L and CMD-D40 (manufactured by Meito Sangyo Co., Ltd.), which are carboxymethyl dextrans, carboxymethylcellulose sodium (manufactured by Wako Pure Chemical Industries, Ltd.), and sodium alginate (manufactured by Wako Pure Chemical Industries, Ltd.), and the like.

As the polymer having a carboxyl group, a polysaccharide having a carboxyl group is preferred and carboxymethyl dextran is more preferable.

The molecular weight of the polymer containing a carboxyl group used in the present invention is not particularly limited. The weight average molecular weight is preferably from 1,000 to 5,000,000, more preferably from 10,000 to 2,000,000, and further more preferably from 100,000 to 1,000,000. When the weight average molecular weight is smaller than the above described range, the amount of a physiologically active substance immobilized becomes small. When the weight average molecular weight is larger than the above described range, it causes a high solution viscosity, and it thereby becomes hard to deal with it.

The hydrophilic polymer as described above may be immobilized to the substrate via the self-assembled monolayer or the hydrophobic polymer as described in this specification, or may directly be formed on the substrate from a monomer-containing solution. In addition, the hydrophilic polymer may be cross-linked. Cross-linking of the hydrophilic polymer is obvious to those skilled in the art.

The polymer having a carboxyl group is activated by a publicly known method using, for example, 1-(3-dimethxylaminopropyl)-3-ethylcarbodiimide (EDC) which is a water-soluble carbodiimide and N-hydroxysuccinimide (NHS) and thus the physiologically active substance having an amino group may be immobilized. To activate the carboxylic acid the method disclosed in JP-A No. 2006-58071, paragraphs [0011] to [0022] (i.e., a method in which a carboxyl group present on the surface of a substrate is activated by using a compound having a specific structure selected from among an uronium salt, a phosphonium salt and a triazine derivative to thereby form a carboxylamide group), or the method described in JP-A No.2006-90781, paragraphs [0011] to [0019] (i.e., a method in which a carboxyl group present on the surface of a substrate is activated by using a carbodiimide derivative or its salt, then esterified with a compound selected from a nitrogen-containing hetero aromatic compound having hydroxyl group, a phenol derivative having an electron-drawing group or an aromatic compound having thiol group and then reacted with an amine to thereby form a carboxylamide group) may be preferably applied. The polymer having a carboxyl group having been activated by such a method is reacted with a substrate having an amino group. Thus, the biosensor according to the invention may be produced.
As a method of activating the polymer having a carboxyl group, there has been known a method wherein a nitrogen-containing compound is used.

In the invention, it is preferable that the polymer having a carboxyl group having been activated and esterified is used in the form of a film to react with the substrate. As a method for forming a thin film on a substrate, known methods can be used. Specific examples of such methods that can be used include an extrusion coating method, a curtain coating method, a casting method, a screen printing method, a spin coating method, a spray coating method, a slide bead coating method, a slit and spin method, a slit coating method, a dye coating method, a dip coating method, a knife coating method, a blade coating method, a flow coating method, a roll coating method, a wire-bar coating method, and a transfer printing method, and the like. These methods for forming a thin film are described in "Progress in Coating Technology (Coating Gijutsu no Shinpo)" written by Yuji Harazaki, Sogo Gyutsu Center (1988); "Coating Technology (Coating Gijutsu)" Technical Information Institute Co., Ltd. (1999); "Aqueous Coating Technology (Suisei Coating no Gijutsu)" CMC (2001); "Evolving Organic Thin Film: Edition for Deposition (Shinka-suru Organic Thin Film: Seimaku hen)" Sumibe Techno Research Co., Ltd. (2004); "Polymer Surface Processing Technology (Polymer Hyomen Kako Gaku)" written by Akira Iwarnori, Gihodo Shuppan Co., Ltd. (2005); and the like. As the method for forming a thin film on a substrate of the present invention, a spray coating method or a spin coating method is preferable. Further, a spin coating method is more preferable. This is because it allows a coating film having a controlled film thickness to be readily produced.

In the biosensor according to the invention, the physiologically active substance is immobilized to the substrate having the metal film to detect the interaction between the physiologically active substance on the substrate and a target substance to be measured in a sample.
A physiologically active substance immobilized on the surface for the biosensor of the present invention is not particularly limited, as long as it interacts with a measurement target. Examples of such a substance may include an immune protein, an enzyme, a microorganism, nucleic acid, a low molecular weight organic compound, a nonimmune protein, an immunoglobulin-binding protein, a sugar-binding protein, a sugar chain recognizing sugar, fatty acid or fatty acid ester, and polypeptide or oligopeptide having a ligand-binding ability.

A physiologically active substance immobilized is not particularly limited, as long as it interacts with a measurement target. Examples of such a substance may include an immune protein, an enzyme, a microorganism, a nucleic acid, a low molecular weight organic compound, a non-immune protein, an immunoglobulin-binding protein, a sugar-binding protein, a sugar chain recognizing sugar, fatty acid or fatty acid ester, and polypeptide or oligopeptide having a ligand-binding ability.

Examples of an immune protein may include an antibody whose antigen is a measurement target, and a hapten. Examples of such an antibody may include various immunoglobulins such as IgG, IgM, IgA, IgE or IgD. More specifically, when a measurement target is human serum albumin, an anti-human serum albumin antibody can be used as an antibody. When an antigen is an agricultural chemical, pesticide, methicillin-resistant Staphylococcus aureus, antibiotic, narcotic drug, cocaine, heroin, crack or the like, there can be used, for example, an anti-atrazine antibody, anti-kanamycin antibody, anti-metamphetamine antibody, or antibodies against O antigens 26, 86, 55,111 or 157 among enteropathogenic Escherichia coli.

An enzyme used as a physiologically active substance herein is not particularly limited, as long as it exhibits an activity to a measurement target or substance metabolized from the measurement target. Various enzymes such as oxidoreductase, hydrolase, isomerase, lyase or synthetase can be used. More specifically, when a measurement target is glucose, glucose oxidase may be used, and when a measurement target is cholesterol, cholesterol oxidase may be used. Moreover, when a measurement target is an agricultural chemical, pesticide, methicillin-resistant Staphylococcus aureus, antibiotic, narcotic drug, cocaine, heroin, crack or the like, enzymes such as acetylcholine esterase, catecholamine esterase, noradrenalin esterase or dopamine esterase, which show a specific reaction with a substance metabolized from the above measurement target, can be used.

A microorganism used as a physiologically active substance herein is not particularly limited, and various microorganisms such as Escherichia coli can be used.
As nucleic acid, those complementarily hybridizing with nucleic acid as a measurement target can be used. Either DNA (including cDNA) or RNA can be used as nucleic acid. The type of DNA is not particularly limited, and any of native DNA, recombinant DNA produced by gene recombination and chemically synthesized DNA may be used.
As a low molecular weight organic compound, any given compound that can be synthesized by a common method of synthesizing an organic compound can be used.

A nonimmune protein used herein is not particularly limited, and examples of such a nonimmune protein may include avidin (streptoavidin), biotin, and a receptor.
Examples of an immunoglobulin-binding protein used herein may include protein A, protein G, and a rheumatoid factor (RF).
As a sugar-binding protein, for example, lectin is used.
Examples of fatty acid or fatty acid ester may include stearic acid, arachidic acid, behenic acid, ethyl stearate, ethyl arachidate, and ethyl behenate.

The physiologically active substance is supplied to the substrate as a physiologically active substance-containing solution prepared by dissolving in an appropriate dissolving liquid. The concentration of the physiologically active substance-containing solution may properly be changed depending upon kind or molecular weight of the target physiologically active substance and the like, but may be generally from 0.001 mg/ml to 10 mg/ml.

The biosensor wherein the physiologically active substance is immobilized as described above can be used for detecting and/or measuring a substance which interact with the physiologically active substance.

A biosensor for surface plasmon resonance is a biosensor used for a surface plasmon resonance biosensor, meaning a member comprising a portion for transmitting and reflecting light emitted from the sensor and a portion for immobilizing a physiologically active substance. It may be fixed to the main body of the sensor or may be detachable.

The surface plasmon resonance phenomenon occurs due to the fact that the intensity of monochromatic light reflected from the border between an optically transparent substance such as glass and a metal thin film layer depends on the refractive index of a sample located on the outgoing side of the metal. Accordingly, the sample can be analyzed by measuring the intensity of reflected monochromatic light.

A device using a system known as the Kretschmann configuration is an example of a surface plasmon measurement device for analyzing the properties of a substance to be measured using a phenomenon whereby a surface plasmon is excited with a lightwave (for example, Japanese Patent Laid-Open No. 6-167443). The surface plasmon measurement device using the above system basically comprises a dielectric block formed in a prism state, a metal film that is formed on a face of the dielectric block and comes into contact with a measured substance such as a sample solution, a light source for generating a light beam, an optical system for allowing the above light beam to enter the dielectric block at various angles so that total reflection conditions can be obtained at the interface between the dielectric block and the metal film, and a light-detecting means for detecting the state of surface plasmon resonance, that is, the state of attenuated total reflection, by measuring the intensity of the light beam totally reflected at the above interface.

In order to achieve various incident angles as described above, a relatively thin light beam may be caused to enter the above interface while changing an incident angle. Otherwise, a relatively thick light beam may be caused to enter the above interface in a state of convergent light or divergent light, so that the light beam contains components that have entered therein at various angles. In the former case, the light beam whose reflection angle changes depending on the change of the incident angle of the entered light beam can be detected with a small photodetector moving in synchronization with the change of the above reflection angle, or it can also be detected with an area sensor extending along the direction in which the reflection angle is changed. In the latter case, the light beam can be detected with an area sensor extending to a direction capable of receiving all the light beams reflected at various reflection angles.

As described in Japanese Patent Laid-Open No. 11-326194, a light-detecting means in the form of an array is considered to be used for the above type of surface plasmon measurement device in order to measure the attenuated total reflection angle (θSP) with high precision and in a large dynamic range. This light-detecting means comprises multiple photo acceptance units that are arranged in a certain direction, that is, a direction in which different photo acceptance units receive the components of light beams that are totally reflected at various reflection angles at the above interface.

In the above case, there is established a differentiating means for differentiating a photodetection signal outputted from each photo acceptance unit in the above array-form light-detecting means with regard to the direction in which the photo acceptance unit is arranged. An attenuated total reflection angle (θSP) is then specified based on the derivative value outputted from the differentiating means, so that properties associated with the refractive index of a measured substance are determined in many cases.

The above-described surface plasmon measurement device may be used in random screening to discover a specific substance (a target substance) binding to a desired sensing substance (a physiologically active substance) in the field of research for development of new drugs or the like. In this case, a sensing substance is immobilized as the above-described measured substance on the above metal thin film layer according to the invention, and a sample solution obtained by dissolving various types of test substance in a solvent is added to the sensing substance. Thereafter, the above-described attenuated total reflection angle (θSP) is measured periodically when a certain period of time has elapsed.

If the test substance (a candidate substance) contained in the sample solution is bound to the sensing substance, the refractive index of the sensing substance is changed by this binding over time. Accordingly, the above attenuated total reflection angle (θSP) is measured periodically after the elapse of a certain time, and it is determined whether or not a change has occurred in the above attenuated total reflection angle (θSP), so that a binding state between the test substance and the sensing substance is measured. Based on the results, it can be evaluated whether or not the candidate substance is a specific substance binding to the sensing substance. Examples of such a combination between a specific substance and a sensing substance may include an antigen and an antibody, and an antibody and an antibody. More specifically, a rabbit anti-human IgG antibody is immobilized as a sensing substance on the surface of a thin film layer, and a human IgG antibody is used as a specific substance.

In order to measure a binding state between a candidate substance and a sensing substance, it is not always necessary to detect the angle itself of an attenuated total reflection angle (θSP), For example, a sample solution may be added to a sensing substance, and the amount of an attenuated total reflection angle (θSP), changed thereby may be measured, so that the binding state can be measured based on the magnitude by which the angle has changed. When the above-described array-form light-detecting means and differentiating means are applied to a measurement device using attenuated total reflection, the amount by which a derivative value has changed reflects the amount by which the attenuated total reflection angle (θSP), has changed. Accordingly, based on the amount by which the derivative value has changed, a binding state between a sensing substance and a test substance can be measured (Japanese Patent Application No. 2003-17694 filed by the present applicant). In a measuring method and a measurement device using such attenuated total reflection, a sample solution consisting of a solvent and a test substance is added dropwise to a cup- or petri dish-shaped measurement chip wherein a sensing substance is immobilized on a thin film layer previously formed at the bottom, and then, the above-described amount by which an attenuated total reflection angle (θSP), has changed is measured.

In the case of using the biosensor according to the invention in the screening as described above, it may be a biosensor that comprises, for example, a detection unit comprising the metal film as described above and the physiologically active substance which is provided on the metal film surface as a sensing substance, a supply unit for supplying a candidate substance to the detection unit, and a screening evaluation unit that evaluates whether the candidate substrate is a target substrate to be screened or whether or not of the binding of the sensing substance and the candidate substance bind together.
In this case, the screening evaluation unit evaluates whether or not the candidate substance is the target substance to be screened based on, for example, a change in refractive index.

In the case of using the biosensor of the invention for surface plasmon resonance analysis, the biosensor may be used as part of a surface plasmon measurement apparatus known in the art.
With respect to storage after the production, it is preferable that the biosensor according to the invention is quickly enclosed in, for example, a nitrogen atmosphere before using. To enclose it in the nitrogen atmosphere, it may be preferably stored in a moisture proof film having a moisture proof performance with a water permeability of 10⁻²g/m² at 40°C 90%RH, or less.

The metal film in the biosensor according to the invention may be fixed to a dielectric block of the measurement unit and integrated as a measurement chip that may replaceable. An example thereof will be described below.

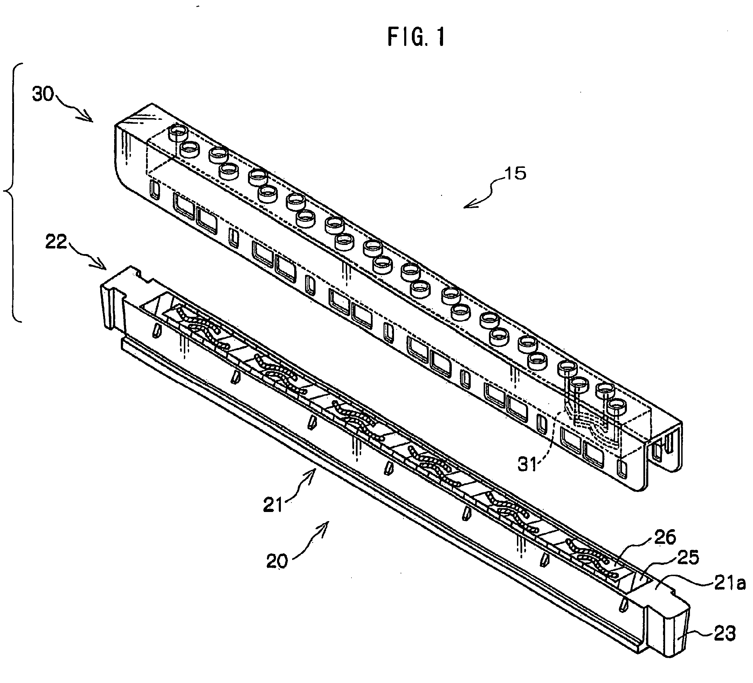

Fig. 1 is an exploded perspective view of a measurement chip 15 for use in measurement utilizing SPR. The measurement chip 15 is constituted by the total reflecting prism 20 which is a transparent dielectric body and the flow channel member 30 attached onto the total reflecting prism 20. On the flow channel member 30 are provided plural flow channels 31 in the longitudinal direction. Additionally, the bottom shape of each flow channel 31 is not particularly limited as long as each of the flow channels 31 has a cross-section as definite as possible. For example, the bottom shape may be a straight linear shape, a meandering shape, or an arc shape, and the like.

The total reflecting prism 20 is constituted by the prism body 21 formed in a long, trapezoidal pillar form, a gripper part 22 provided on one end of the prism body 21, and a projecting part 23 provided on the other end of the prism body 21. This total reflecting prism 20 is formed by, for example, molding according to the extrusion method, wherein the prism body 21, the gripper part 22, and the projecting portion 23 are molded in an integrated manner.

The prism body 21 has an almost trapezoidal cross-section wherein the upper side is longer than the lower side, and collects light irradiated from the side of the bottom surface on a upper surface 21a. On the upper surface 21a of the prism body 21, the metal film 25 for exciting SPR is provided. The metal film 25 has a rectangular shape in such a way that it faces each flow channel 31 of the flow channel member 30, and is formed by, for example, vapor deposition.

On the metal film 25, the polymer layer 26 is provided. The polymer layer 26 has a binding group for immobilizing a physiologically active substance. The physiologically active substance is immobilized to the metal film 25 via the polymer layer 26.
As described above the polymer layer 26 is a layer for immobilizing the physiologically active substance and can be constituted by self-assembled monolayer-forming molecules, a hydrophilic polymer, a hydrophobic polymer or a combination thereof. Among these, the hydrophilic polymer is preferably used for the polymer layer 26. According to a particularly preferred embodiment, it may be constituted by a combination of self-assembled monolayer-forming molecules and a hydrophilic polymer.

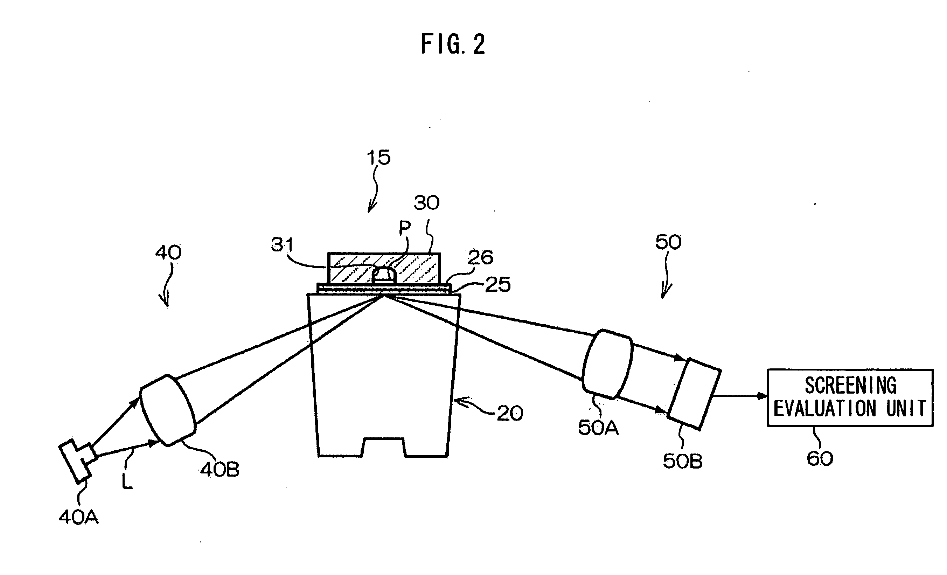

Fig. 2 is a schematic view showing a biosensor 10 for screening which is an example of a biosensor having the measurement chip 15 as described above.
The biosensor 10 comprises a measurement chip 15 on which a physiologically active substance P as a sensing substance on a metal film 25, a light emitting unit 40 which emits light toward the measurement chip 15, a light receiving unit 50 which receives reflected light from the measurement chip 15, and a screening evaluation unit 60 which conducts screening based on the light data obtained from the light receiving unit 50.
The light emitting unit 40 comprises a light source 40A emitting light beams L and a lens unit 40B which allows the light beams L to enter a total reflecting prism 20 at various angles so that the total reflection conditions can be established at the interface between the total reflecting prism 20 and the metal film 25.

On the measurement chip 15, a channel 31 as a supply unit is formed by a channel member 30 so as to supply a candidate substance to be screened in a sample to the physiologically active substance P having been immobilized on the metal film 25 as described above. The supply unit may be provided with a liquid-feeding system (not shown in the drawing) for supplying the sample solution to the channel 31.
The light receiving unit 50 comprises a lens unit 50A and a light detection unit 50B. The lens unit 50A converts the light beams L totally reflected at the above-described interface between the total reflecting prism 20 and the metal film 25 of the measurement chip 15 into parallel light. The light detection unit 50B detects the light beams L having been parallelized by the lens unit 50A. As the light detection unit 50B, A phtodiode array, a CCD and the like may be used.

The screening evaluation unit 60 is connected to the light receiving unit 50 and calculates the refractive index based on the light detected by the light receiving unit 50 as described above and, based on this refractive index, determines the interacting properties of the physiologically active substance P on the metal film 25 in the measurement chip 15 and the candidate substance in the sample, thereby judging whether the candidate substance is the target substance to be screened or not. The judgment may be altered depending on the purpose of the screen. For example, a substance interacting with the physiologically active substance P may be extracted as a target substance from candidate substances. Alternatively, a substance not interacting with the physiologically active substance P may be extracted as a target substance from candidate substances.

The biosensor of the present invention may be used as a biosensor, which has a waveguide structure on the surface of a substrate, for example, and which detects refractive index changes using such a waveguide. In this case, the waveguide structure on the substrate surface has a diffraction grating, and in some cases, an additional layer. This waveguide structure is a planar waveguide body comprising a thin dielectric layer. Light gathered to the waveguide body form is introduced into such a thin layer by total internal reflection. The transmission velocity of the thus introduced light wave (hereinafter referred to as "mode") is indicated as a C/N value where C indicates the velocity of light in a vacuum, and N indicates an effective refractive index of the mode introduced into the waveguide body. An effective refractive index N is determined based on the structure of the waveguide body on one face, and is determined based on the refractive index of a medium adjacent to the thin waveguide layer on the other face. Conduction of a light wave is carried out not only in a thin planar layer, but also by another waveguide structure, and in particular, by a stripped waveguide body. In such a case, the waveguide structure is processed into the shape of a stripped film. It is an important factor for a biosensor that changes in effective refractive indexes N are generated as a result of changes in the medium adjacent to the waveguide layer, and changes in the refractive index and thickness of the waveguide layer itself or an additional layer adjacent to the waveguide layer.

The structure of a biosensor of this system is described, for example, in page 4, line 48 to page 14, line 15, and FIGS. 1 to 8 of JP-B No. 6-27703 (1994), and column 6, line 31 to column 7, line 47, and FIGS 9A and 9B of US Patent No. 6,829,073.

In another embodiment, it is also possible to adopt a structure whereby an array of diffraction grating waveguides is incorporated into wells of a microplate (JP-A No. 2007-501432, FIG 1). That is, if such diffraction grating waveguides are aligned in the form of an array at the bottoms of wells of a microplate, the screening of a drug or chemical substance may be carried out at a high throughput.

### [Examples]

### [Example 1]

### (Preparation of sample)

This Example relates to the production of sensor chips for immobilizing a protein.

### (1-1) Preparation of sample 1 (Comparative Example 1)

On the surface of a prism made of a cycloolefin polymer employed in the sensor stick of AP3000 system (manufactured by FUJIFILM Corporation) that is a biosensor system using surface plasmon resonance, a metal film having a thickness of 50 nm was formed by using an argon sputter deposition device.

### (1-2) Preparation of sample 2 (Comparative Example 2)

A silver film having a thickness of 50 nm was formed in the same manner as in sample 1 except that only silver was used as the target in the sputter deposition.

### (1-3) Preparation of samples 3 to 8

Alloy films each having a thickness of 50 nm were formed in the same manner as in samples 1 and 2 except that the alloys listed in Table 1 was used as the targets in the sputter deposition. The film thickness was controlled by checking up the sputter conditions with the use of an ellipsometer.

**[Table 1]**

| Sample no. | Composition | |
|---|---|---|
| Sample 1 | Au 100 | Comparative Example |
| Sample 2 | Ag 100 | Comparative Example |
| Sample 3 | Ag 98.1, Cu 0.9, Au 1.0 | |
| Sample 4 | Ag 98.4, Nd 0.7, Cu 0.9 | |
| Sample 5 | Ag 98.3, Nd 0.7, Au 1.0 | |
| Sample 6 | Ag 99.5, Bi 0.5 | |
| Sample 7 | Ag 95, Au 5 | |
| Sample 8 | Ag 90, Au 10 | |

### (1-4) Preparation of samples 9 to 16

On the metal films formed by sputter deposition in the same manner as in samples 1 to 8, Si was sputter deposited under an oxygen atmosphere without breaking the vacuum to form SiO₂ films each having a thickness of 20 nm. Thus, sensor stick samples 9 to 16 having the SiO₂ films as a protective layer were obtained.

### (2-1) Production of substrate having OH group

The above-described sensor stick samples 1 to 16 having the metal films and SiO₂ films were surface-treated in the following manner.
Each sensor stick was treated with UV ozone for 12 minutes and then immersed in an ethanol/water mixture (80/20) containing 5.0 mM 16-hydroxyhexadecanethiol (manufactured by Frontier Scientific) dissolved therein. After incubating in a shaking incubator at 40°C for 20 minutes, the sample was washed 5 times with water, 5 times with 50 ml of ethanol/water (80/20) and 5 times with 50 ml of water.

### (2-2) Treatment with epichlorohydrin

The sample treated as described above was immersed in a mixture of 20 ml of 4 M sodium hydroxide, 20 ml of diethylene glycol dimethyl ether and 2.0 ml of epichlorohydrin. After reacting in a shaking incubator at 25°C for 4 hours, the sample was washed twice with 50 ml of ethanol and 5 times with 50 ml of water.

### (2-3) Treatment with dextran

The sample treated as described above was immersed in a mixture of 40.5 ml of water, 13.5 g of dextran (T500, manufactured by Pharmacia) and 4.5 ml of 1 M sodium hydroxide. After reacting in a shaking incubator at 25°C for 20 hours, the sample was washed 15 times with 50 ml of water at 50°C.

### (2-4) Treatment with bromoacetic acid

The sample treated as described above was immersed in a mixture of 3.5 g of bromoacetic acid and 27 g of a 2M sodium hydroxide solution. After reacting in a shaking incubator at 28°C for 16 hours, the sample was washed with water. After reacting with the bromoacetic acid solution again for 16 hours, the sample was washed with water.

### (3) Production of substrate having amino group

A sensor stick having a metal film formed thereon was treated with UV ozone for 12 minutes and then reacted in a solution prepared by dissolving 45 µmol of 11-hydroxy-1-undecanethiol (manufactured by Aldrich) and 4 µmol of 16-mercaptohexadecanoic acid (manufactured by Aldrich) in 8 ml of ethanol and 2 ml of ultrapure water at 40°C for 1 hour. Then, the stick was washed once with ethanol and once with ultrapure water. To the sample treated as described above, 100 µl of a mixture of EDC (0.4 M) with NHS (0.1 M) was added dropwise and then the sample was reacted at room temperature for 15 minutes. After thus activating, the sample was washed once with ultrapure water. To the sample having been treated as described above, 50 µl of 1,2-bis(aminoethoxy)ethane was added dropwise and the sample was reacted at room temperature for 1 hour. Then, the sample was washed once with ultrapure water.

### (4-1) Activation and esterification of CMD

After dissolving CMD (molecular weight: 1,000,000, manufactured by Meito Sangyo, Col, Ltd.) in ultrapure water to give a concentration of 0.5% by weight, a mixture of EDC (1-(3-dimethylaminopropyl)-3-ethylcarbodiimide) (0.4 M) and NHS (N-hydroxysuccinimide) (0.1 M) in an amount such that 2% of carboxyl groups would be activated if the total amount was reacted, was added, and the resultant mixture was stirred at room temperature for 5 minutes.

### (4-2) Binding of activated and esterified CMD to substrate

Onto the substrate produced in (3), 200 µl of the activated and esterified CMD solution prepared in (4-1) was added dropwise. By spin-coating at 7000 rpm for 45 seconds, an activated and esterified carboxymethyldextran film was formed on the treated sample having amino group. After reacting at room temperature for 1 hour, the sample was washed once with 0.1 N NaOH and once with ultrapure water. Thus, sensor stick samples 17 to 32 further having an immobilization film were obtained.

### [Example 2]

### (Evaluation of sensitivity and surface plasmon absorption line)

On the next day of the production, the samples 1 to 16 produced in Example 1 were mounted to AP3000 (i.e., a system using surface plasmon resonance manufactured by FUJIFILM Corporation that had been partly modified) and the half widths of resonance absorption lines were relatively evaluated. Table 2 shows the obtained results. The half-widths are expressed in relative values calculated by referring the value of sample 1 using a gold film as to 100. The light source employed in the evaluation was a super luminescent diode of 830 nm in wavelength.

**[Table 2]**

| Sample No. | Half-width value | Protective layer | Remarks |
|---|---|---|---|
| Sample 1 | 100 | - | Comparative Example |
| Sample 2 | 76 | - | Comparative Example |
| Sample 3 | 68 | - | |
| Sample 4 | 65 | - | |
| Sample 5 | 60 | - | |
| Sample 6 | 65 | - | |
| Sample 7 | 66 | - | |
| Sample 8 | 68 | - | |
| Sample 9 | 115 | SiO₂ | Comparative Example |
| Sample 10 | 85 | SiO₂ | Comparative Example |
| Sample 11 | 73 | SiO₂ | |
| Sample 12 | 70 | SiO₂ | |
| Sample 13 | 66 | SiO₂ | |
| Sample 14 | 69 | SiO₂ | |
| Sample 15 | 69 | SiO₂ | |
| Sample 16 | 72 | SiO₂ | |

As Table 2 clearly shows, it was confirmed that the sensor sticks according to the invention showed narrower half-widths than comparative samples 1 and 2 comprising a gold film and, therefore, had higher detection sensitivities.

### [Example 3]

### (Evaluation of durability)

The sensor sticks produced in Example 1 were stored in an environment of 30°C 60% for 3 days. Then, the same experiment as in Example 2 was conducted.
Comparative samples 2 and 10 showed remarkable decreases in the resonance absorption line intensity and, therefore, could not be used in the measurement. Samples 3 to 8 and 11 to 16 according to the invention maintained narrow resonance absorption line half widths and extremely regulated decreases in the peak intensity. This tendency was particularly remarkable in samples 11 to 16.

**[Table 3]**

| Sample No. | Half-width value | Half-width value after storage | Remarks |
|---|---|---|---|
| Sample 1 | 100 | 102 | Comparative Example |
| Sample 2 | 76 | - | Comparative Example |
| Sample 3 | 68 | 80 | |
| Sample 4 | 65 | 75 | |
| Sample 5 | 60 | 70 | |
| Sample 6 | 65 | 85 | |
| Sample 7 | 66 | 85 | |
| Sample 8 | 68 | 80 | |
| Sample 9 | 115 | 116 | Comparative Example |
| Sample 10 | 85 | - | Comparative Example |
| Sample 11 | 73 | 79 | |
| Sample 12 | 70 | 73 | |
| Sample 13 | 66 | 69 | |
| Sample 14 | 69 | 74 | |
| Sample 15 | 69 | 80 | |
| Sample 16 | 72 | 78 | |

### [Example 4]

### (Performance after long storage)

Immediately after the production, sensor stick samples 3 to 8 and 11 to 16 produced in Example 1 were enclosed in a nitrogen atmosphere, etc. To enclose them in the nitrogen atmosphere, use was made of a moisture proof film having a moisture proof performance with a water permeability of 10⁻²g/m² at 40°C 90%. After storing at 6°C for 6 months, the same experiment as in Example 2 was conducted.
Then, samples 3 to 8 and 11 to 16 according to the invention showed almost the same resonance absorption lines as immediately after the production.

### [Example 5]

This Example relates to the immobilization of a protein to sensor stick samples 17 to 32 obtained in Example 1. CA (carbonic anhydrase, manufactured by SIGMA) was used as the protein. By comparing with markers (Broad pI kit, pH 3.5-9.3; manufactured by Amersham Bioscience) simultaneously measured in an electrophoresis test using AE-8150 (manufactured by ATTO), it was confirmed that the employed CA had an isoelectric point of about 5.8. 1 mg of CA was dissolved in 1 ml of HBS-EP buffer (manufactured by Biacore, 0.01 M of HEPES (pH 7.4), 0.15 M of NaCl, 0.005% of a surfactant P20, 3 mM of EDTA). 10 µl of the obtained solution was taken up and 90 µl of acetate buffer (manufactured by Biacore, pH 5.0) was added thereto to give a 0.1 mg/ml CA solution (pH 5.0, 0.1 mg/ml).

Samples 17 to 32 produced in Example 1 were mounted to AP3000 (i.e., a system using surface plasmon resonance manufactured by FUJIFILM Corporation). Then, an aqueous solution containing 0.4 M of EDC and 0.1 M of NHS, the CA solution (pH 5.0, 0.1 mg/ml) and an ethanolamine solution (manufactured by Biacore) were flown each for 5 minutes followed by flowing 10 mM of NaOH twice each for 1 minute. Then the immobilization was examined. As a running buffer, use was made of HBS-N buffer (manufactured by Biacore, 0.01 M of HEPES (pH 7.4), 0.15 M of NaCl).

Thus, it has been proved that the invention makes it possible to easily produce a CMD-bound surface having a larger amount of the protein immobilized thereon than a commercially available CMD-bound surface or the CMD-bound surface produced by the existing method.

### [Example 6]

This Example relates to the pre-concentration immobilization (concentration, or immortalization) of a protein to the sensor chips obtained in Example 1. BSA (bovine serum albumin, manufactured by SIGMA) was used as the protein. By comparing with markers (Broad pI kit, pH 3.5-9.3; manufactured by Amersham Bioscience) simultaneously measured in an electrophoresis test using AE-8150 (manufactured by ATTO), it was confirmed that the employed BSA had an isoelectric point of about 6.1. 1 mg of BSA was dissolved in 1 ml of HBS-EP buffer (manufactured by Biacore, pH 7.4). 10 µg of the obtained solution was taken up and 90 µl of acetate buffer (manufactured by Biacore, pH 5.0) was added thereto to give a 0.1 mg/ml BSA solution (pH 5.0, 0.1 mg/ml).

Samples 3 to 8 and 11 to 16 produced in Example 1 were mounted to AP3000 (i.e., a system using surface plasmon resonance manufactured by FUJIFILM Corporation) and the BSA solution (pH 5.0, 0.1 mg/ml) was flown for 5 minutes. Then, the pre-concentration was examined and favorable results were obtained.

It will be obvious to those having skill in the art that many changes may be made in the above-described details of the preferred embodiments of the present invention. The scope of the invention, therefore, should be determined by the following claims.

## Claims

1. A biosensor for detecting the interaction of a physiologically active substance on a substrate based on surface plasmon resonance, wherein the substrate has a metal film (25) comprising:
90% by mol or more and less than 99.95% by mol of a first metal element which is Ag; and
0.05% by mol or more and less than 10% by mol of a second metal element, which is at least one of Bi, Nd or Sb.

2. The biosensor according to claim 1, wherein the second metal element further comprises at least one selected from a first group consisting of Au, Pt, Cu and Ti.

3. The biosensor according to claims 1 or 2, wherein the second metal element comprises 0.1 % by mol to 2% by mol of at least one selected from a second group consisting of Bi, Nd and Sb.

4. The biosensor according to any one of claims 1 to 3, wherein the metal film (25) has a thickness of 0.1 nm to 500 nm.

5. The biosensor according to any one of claims 1 to 4, wherein a protective layer comprising at least one of a metal oxide and a metal nitride is provided on the metal film.

6. The biosensor according to any one of claims 1 to 5, wherein a layer of an alkanethiol, having at least one of an amino group or a hydroxyl group, is provided on the metal film (25).

7. The biosensor according to any one of claims 1 to 6, further comprising a hydrophilic polymer layer (26) provided on the metal film (25).

8. The biosensor according to any one of claims 1 to 7, wherein the metal film is provided on a plastic substrate.

9. The biosensor according to any one of claims 1 to 8, comprising:
a detection unit (15) comprising the metal film (25) and the physiologically active substance (P) which is provided on the metal film (25) surface as a sensing substance;
a supply unit for supplying a candidate substance to the detection unit (15); and
a screening evaluation unit that evaluates whether the candidate substrate is a target substrate to be screened or not based on whether or not the sensing substance and the candidate substance bind together.

## Patentansprüche

1. Biosensor zum Ermitteln der Interaktion einer physiologisch aktiven Substanz auf einem Substrat auf der Basis der Oberflächenplasmonresonanz, worin das Substrat einen Metallfilm (25) hat, umfassend:
90 Mol% oder mehr und weniger als 99,95 Mol% eines ersten Metallelementes, das Ag ist, und
0,05 Mol% oder mehr und weniger als 10 Mol% eines zweiten Metallelementes, das zumindest eines von Bi, Nd oder Sb ist.

2. Biosensor nach Anspruch 1, worin das zweite Metallelement weiterhin zumindest eines umfasst, ausgewählt aus einer ersten Gruppe, bestehend aus Au, Pt, Cu und Ti.

3. Biosensor nach Anspruch 1 oder 2, worin das zweite Metallelement 0,1 Mol% bis 2 Mol% von zumindest einem umfasst, ausgewählt aus einer zweiten Gruppe, bestehend aus Bi, Nd und Sb.

4. Biosensor nach einem der Ansprüche 1 bis 3, worin der Metallfilm (25) eine Dicke von 0,1 nm bis 500 nm hat.

5. Biosensor nach einem der Ansprüche 1 bis 4, worin eine Schutzschicht, umfassend zumindest eines von einem Metalloxid und einem Metallnitrid, auf dem Metallfilm vorgesehen ist.

6. Biosensor nach einem der Ansprüche 1 bis 5, worin eine Schicht aus einem Alkanthiol mit zumindest einer von einer Aminogruppe oder einer Hydroxylgruppe, auf dem Metallfilm (25) vorgesehen ist.

7. Biosensor nach einem der Ansprüche 1 bis 6, weiterhin umfassend eine hydrophile Polymerschicht (26), die auf dem Metallfilm (25) vorgesehen ist.

8. Biosensor nach einem der Ansprüche 1 bis 7, worin der Metallfilm auf einem Kunststoffsubstrat vorgesehen ist.

9. Biosensor nach einem der Ansprüche 1 bis 8, umfassend:
eine Detektionseinheit (15), umfassend den Metallfilm (25) und die physiologisch aktive Substanz (P), die auf der Oberfläche des Metallfilms (25) als Abtastsubstanz vorgesehen ist;
eine Zuführeinrichtung zum Zuführen einer Kandidatensubstanz zur Detektionseinheit (15) und
eine Screening-Bewertungseinheit, die bewertet, ob die Kandidatensubstanz eine Zielsubstanz, die gescreent werden soll, ist oder nicht, auf der Basis, ob die Abtastsubstanz und die Kandidatensubstanz zusammen binden oder nicht.

## Revendications

1. Biocapteur pour détecter l'interaction d'une substance physiologiquement active sur un substrat basé sur une résonance de plasmon en surface, dans lequel le substrat présente un film de métal (25) comprenant :
90 % en mole ou plus et moins de 99,95 % en mole d'un premier élément de métal qui est Ag ; et
0,05 % en mole ou plus et moins de 10 % en mole d'un second élément de métal qui est au moins un parmi Bi, Nd ou Sb.

2. Biocapteur selon la revendication 1, dans lequel le second élément de métal comprend de plus au moins un choisi dans un premier groupe constitué de Au, Pt, Cu et Ti.

3. Biocapteur selon la revendication 1 ou 2, dans lequel le second élément de métal comprend de 0,1 % en mole à 2 % en mole d'au moins un élément choisi dans un second groupe constitué de Bi, Nd et Sb.

4. Biocapteur selon l'une quelconque des revendications 1 à 3, dans lequel le film de métal (25) présente une épaisseur de 0,1 nm à 500 nm.

5. Biocapteur selon l'une quelconque des revendications 1 à 4, dans lequel une couche protectrice comprenant au moins un parmi un oxyde de métal et un nitrure de métal est fournie sur le film de métal.

6. Biocapteur selon l'une quelconque des revendications 1 à 5, dans lequel une couche d'un alcanethiol, présentant au moins un groupe parmi un groupe amino ou un groupe hydroxyle, est fournie sur le film de métal (25).

7. Biocapteur selon l'une quelconque des revendications 1 à 6, comprenant de plus une couche de polymère hydrophile (26) fournie sur le film de métal (25).

8. Biocapteur selon l'une quelconque des revendications 1 à 7, dans lequel le film de métal est fourni sur un substrat en plastique.

9. Biocapteur selon l'une quelconque des revendications 1 à 8, comprenant:
une unité de détection (15) comprenant le film de métal (25) et la substance physiologiquement active (10) qui est fournie sur la surface de film de métal (25) comme une substance de détection;
une unité d'alimentation pour alimenter l'unité de détection (15) en une substance candidate ; et
une unité d'évaluation par balayage qui évalue si le substrat candidat est un substrat cible à balayer ou non selon que la substance de détection et la substance candidate se lient ou non l'une à l'autre.
